# EUROPEAN PATENT SPECIFICATION

(11) **EP 3 113 700 B1**
(45) Date of publication and mention of the grant of the patent: **09.05.2018**
(21) Application number: 15716134.0
(22) Date of filing: 04.03.2015
(51) Int. Cl.: A61B 17/28, A61B 17/42

(54) **TENACULUM FOR LAPAROSCOPIC HYSTERECTOMY**
TENAKULUMZANGE FÜR LAPAROSKOPISCHE HYSTEREKTOMIE
TENACULUM POUR HYSTÉRECTOMIE LAPAROSCOPIQUE

(30) Priority: 05.03.2014 IT RM20140103
(43) Date of publication of application: 11.01.2017
(73) Proprietor: Barletta, Francesco, 00182 Roma (IT)
(72) Inventor: VIZZA, Enrico, 00198 Roma (IT); BARLETTA, Francesco, 00182 Roma (IT)
(86) International application number: PCT/IB2015/051578
(87) International publication number: WO 2015/132736

(56) References cited:
- CN-U- 202 184 777
- CN-U- 202 761 400
- CN-U- 202 801 731
- FR-A1- 2 580 166
- US-A- 5 059 198
- US-A- 5 893 878
- US-A1- 2012 203 272

## Description

### Technical Field

The present invention relates to a tenaculum for laparoscopic hysterectomy. It belongs to the technological field of multipurpose surgical steel instruments for use in laparoscopy.

### Background of the art

Currently, in surgery of laparoscopic hysterectomy the delineation of the vaginal edges to be incised, in order to avoid incorrect dissection planes, relies on uterine manipulators, which are expensive and rather complex devices and, sometimes, not easy to be used and applied.

These costs and maneuverability limit the adoption of uterine manipulators in health care facilities with the occasional consequence of not using any instruments and thus increasing the risk of surgical incision in incorrect anatomical sites and, in particularly, in oncological diseases, increasing the risk of diffusion of neoplastic cells due to the invasiveness of the aforesaid manipulators.

The catalog of KLS Martin surgical instruments lists a uterine tenaculum forceps Schroeder, model code 32-622-25. It comprises a first lever and a second lever pivoted between them. The levers have proximal ends provided with finger ring and facing retaining elements to maintain a desired clamping pressure of the human tissue to be grasped. In the distal ends of its two levers the tenaculum has at least a pair of curved substantially facing points.

The forceps described above, if used to hold and pull out the uterus, is not particularly useful to indicate the incision and opening line of the vaginal fundus to a surgeon in the context of a laparoscopic hysterectomy.

Further US 2012/0203272 A1 discloses a tonsil forceps comprising a front lever and a rear lever pivoted in a central position thereof, the front lever and a rear lever having proximal ends provided with a finger ring, and distal ends, each provided with at least one point, the points being curved towards one another and substantially facing. Each of the distal ends of the levers comprises grasping means for securing a tonsil, with each grasping means comprising a palm-like curved portion. The curved portions have inwardly facing projection members located thereon, the projection members being blunt structural features, such as raised ridges, mounds, posts or the like which will abut the sides of the tonsil but not penetrate or cut into its surface when the forceps are closed around the tonsil.

### Disclosure of the invention

One object of the present invention is to provide a forceps for the uterine cervix that allows to raise the vaginal wall from the uterine cervix, just enough to delineate the incision of the vaginal fundus and show at best the edges to be incised in order to adjust the width of the vaginal tract to be excised.

### Brief description of drawings

The principle of operation and the main features of the present invention may be better understood from the detailed description of an embodiment thereof, with reference to the accompanying drawings, in which:

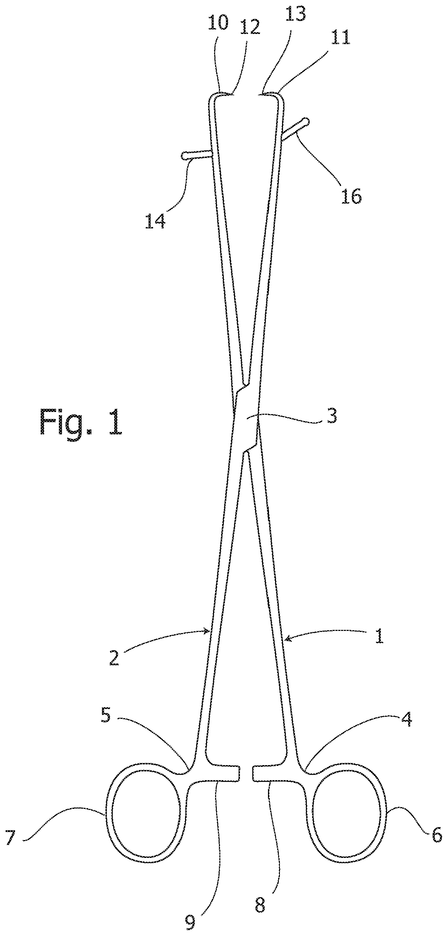

FIG. 1 is a schematic side view of the tenaculum for laparoscopic hysterectomy according to the present invention;

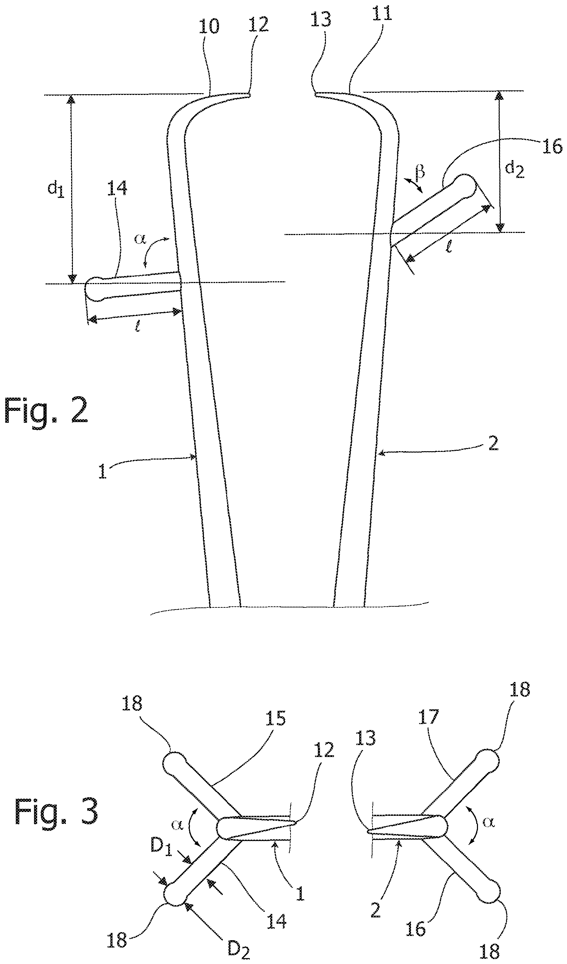

FIG. 2 is an enlarged detail of FIG. 1; and
FIG. 3 is a distal view of a further enlarged part in FIG. 2.

### Description of an embodiment of the invention

The tenaculum for laparoscopic hysterectomy according to the present invention comprises a first lever or front lever 1 and a second lever or rear lever 2 pivotably mounted in a central position 3 thereof. The levers 1, 2 have proximal ends 4, 5 with a finger ring 6, 7 and facing retaining elements 8, 9 in order to maintain a desired clamping pressure of the human tissue to grasp. The distal ends (10, 11), opposite the finger ring 6, 7 of the two levers 1, 2, are curved towards one another with the points (12, 13) facing each other.

According to the invention, the front lever 1 has a first pair of prongs or nebs 14, 15 of length ℓ, projecting outwardly in a V shape symmetrically and orthogonally to the front lever 1, opposite to the rear lever 2. The first pair of prongs 14, 15 is located at a given distance d₁ from the distal end 10, where the front lever 1 curves in the point 12. The front lever 1 with its prongs 14, 15 allows the anterior fornix and the anterior lateral fornices of the vagina to be shown.

The rear lever 2 has a second pair of prongs 16, 17 of length ℓ, projecting outwardly in a V shape symmetrically at an acute angle β with respect to the rear lever 2, opposite to the front lever 1. The second pair of prongs 16, 17 is located at a given distance d₂ from the distal end 11, where the rear lever curves in the point 13. The rear lever 2 with its prongs 16, 17 allows the posterior fornix and the posterior lateral fornices of the vagina to be shown. The acute angle β is between 45 degrees and 55 degrees, preferably about 50 degrees. This angle β is secondary to the average anatomical angle between the axis passing through the uterine body and the one passing through the cervix, usually not less than 40 degrees. The prongs 16, 17, having this angle, can more easily delineate rear the vagina near its insertion with the uterus.

Suitably, the prongs 14, 15 and 16, 17 of the first and second pair of prongs have a cylindrical shape and end with a spherical terminal portion generally indicated as 18. The diameter D₁ of the prongs is preferably 2 mm, and the diameter D₂ of their spherical terminal portions 18 is preferably 2.7 mm.

The length ℓ of the prongs of the two pairs of prongs 14, 15 and 16, 17 is between 7 and 10 mm, and is preferably 8 mm. The prongs 14 and 15 of the first pair of prongs, as well as the prongs 16, 17 of the second pair of prongs form an angle α of 90 degrees between them.

The distance d₁ of the pair of prongs 14, 15 from the distal end 10 of the front lever 1 of the tenaculum is 15-25 mm, preferably 20 mm, and the distance d₂ of the pair of prongs 16, 17 from the distal end 11 of the rear lever 2 of the tenaculum is 10-20 mm, preferably 15 mm.

During an operation of laparoscopic hysterectomy an assistant, after having placed the tenaculum via the vagina on the uterine cervix, pushes the tenaculum towards the inside of the abdominal cavity and gently turns it 180 degrees so that the prongs, placed in the vicinity of the distal end of the tenaculum, raise the vaginal wall in all its circular course enough to indicate to the surgeon the incision and opening line of the vaginal fundus.

The tenaculum according to the present invention allows the vaginal edges to incise to be shown so that the laparoscopic surgeon has also the possibility of adjusting the width of the vaginal tract to remove by applying a higher or lower pressure on the tenaculum towards the inside of the abdominal cavity.

It is understandable that the tenaculum described above combines the ease of use of a forceps that is very familiar to gynecologist surgeons with the low cost of the instrument, which in addition is reusable after sterilization.

It should be obvious that the tenaculum according to the present invention could have a pair of points at the distal end of each lever. In this way the forceps Schroeder BF-59 as presented in the catalog of Bontempi Surgical Instruments would be improved.

## Claims

1. A tenaculum for laparoscopic hysterectomy comprising a front lever (1) and a rear lever (2) pivoted in a central position (3) thereof, the front lever (1) and a rear lever (2) having proximal ends (4, 5) provided with a finger ring (6, 7), and distal ends (10, 11), each provided with at least one point (12, 13), the distal ends (10, 11) being curved towards one another with the points (12, 13) facing each other, **characterized in that**:
said front lever (1) has a first pair of prongs (14, 15) of length ℓ projecting outwardly in a V shape symmetrically and orthogonally to the front lever (1), opposite to the rear lever (2), and at a given distance d₁ from said distal end (10); and
said rear lever (2) has a second pair of prongs (16, 17) of length ℓ projecting outwardly in a V shape symmetrically at an acute angle β with respect to the rear lever (2), opposite to the front lever (1), and at a given distance d₂ from the distal end (11).

2. The tenaculum according to claim 1, wherein said acute angle β formed by said second pair of prongs (16, 17) with respect to the rear lever (2) is between 45 degrees and 55 degrees.

3. The tenaculum according to claim 2, wherein said acute angle β formed by said second pair of prongs (16, 17) with respect to the rear lever (2) is preferably 50 degrees.

4. The tenaculum according to claim 1, wherein the prongs (14, 15 and 16, 17) of the first and second pair of prongs have a cylindrical shape and end with a spherical terminal portion (18).

5. The tenaculum according to claim 4, wherein the prongs (14, 15 and 16, 17) have a diameter D₁ of 2 mm, and their spherical terminal portions (18) have a diameter D₂ of 2.7 mm.

6. The tenaculum according to claim 1, wherein the length ℓ of the prongs is between 7 and 10 mm.

7. The tenaculum according to claim 1, wherein the length ℓ of the prongs is preferably 8 mm.

8. The tenaculum according to claim 1, in which the distance d₁ of the pair of prongs (14, 15) from the distal end (10) of the front lever (1) of the tenaculum is comprised in a range of 15-25 mm.

9. The tenaculum according to claim 8, in which the distance d₁ of the pair of prongs (14, 15) from the distal end (10) of the front lever (1) of the tenaculum is preferably 20 mm.

10. The tenaculum according to claim 1, in which the distance d₂ of the pair of prongs (16, 17) from the distal end (11) of the rear lever (2) of the tenaculum is comprised in a range of 10-20 mm.

11. The tenaculum according to claim 1, in which the distance d₂ of the pair of prongs (16, 17) from the distal end (11) of the rear lever (2) of the tenaculum is preferably 15 mm.

12. The tenaculum according to claim 1, wherein the prong (14) forms an angle α of 90 degrees with the prong (15), and the prong (16) forms an angle α of 90 degrees with the prong (17).

## Patentansprüche

1. Tenakulumzange für laparoskopische Hysterektomie, umfassend einen frontseitigen Hebel (1) und einen rückseitigen Hebel (2), die an einer mittigen Position (3) drehbar gelagert sind, wobei der frontseitige Hebel (1) und ein rückseitiger Hebel (2) proximale Ende (4, 5) aufweisen, die mit einem Fingerring (6, 7) versehen sind, sowie distale Enden (10, 11), die jeweils mit mindestens einem Punkt (12, 13) versehen sind, wobei die distalen Enden (10, 11) zueinander hinführend gekrümmt sind, wobei die Punkte (12, 13) einander zugewandt sind, **dadurch gekennzeichnet ist, dass**
der frontseitige Hebel (1) ein erstes Paar Zinken (14, 15) einer Länge ℓ aufweist, nach außen hervorstehend in einer V-Form, symmetrisch und rechtwinkelig zum frontseitigen Hebel (1), gegenständig zum rückseitigen Hebel (2) und in einem vorgegebenen Abstand d₁ vom distalen Ende (10), und dass
der rückseitige Hebel (2) ein zweites Paar Zinken (16, 17) einer Länge ℓ aufweist, nach außen hervorstehend in einer V-Form, symmetrisch in einem spitzen Winkel β zum rückseitigen Hebel (2), gegenständig zum frontseitigen Hebel (1) und in einem vorgegebenen Abstand d₂ vom distalen Ende (11),

2. Tenakulumzange nach Anspruch 1, wobei der spitze Winkel β, den das zweite Paar zinken (16, 17) zum rückseitigen Hebel (2) bildet, zwischen 45 Grad und 55 Grad beträgt.

3. Tenakulumzange nach Anspruch 2, wobei der spitze Winkel β, den das zweite Paar Zinken (16, 17) zum rückseitigen Hebel (2) bildet, vorzugsweise 50 Grad beträgt.

4. Tenakulumzange nach Anspruch 1, wobei die Zinken (14, 15 und 16, 17) des ersten und zweiten Paars Zinken eine zylindrische Form aufweisen und mit einem kugelförmigen Endabschnitt (18) enden.

5. Tenakulumzange nach Anspruch 4, wobei die Zinken (14, 15 und 16, 17) einen Durchmesser D₁ von 2 mm aufweisen und deren kugelförmigen Endabschnitte (18) einen Durchmesser D₂ von 2,7 mm aufweisen.

6. Tenakulumzange nach Anspruch 1, wobei die Länge ℓ der Zinken zwischen 7 und 10 mm beträgt.

7. Tenakulumzange nach Anspruch 1, wobei die Länge ℓ der Zinken vorzugsweise 8 mm beträgt.

8. Tenakulumzange nach Anspruch 1, wobei der Abstand d₁ des Paars Zinken (14, 15) vom distalen Ende (10) des frontseitigen Hebels (1) der Tenakulumzange in einem Bereich von 15 bis 25 mm enthalten ist.

9. Tenakulumzange nach Anspruch 8, wobei der Abstand d₁ des Paars Zinken (14, 15) vom distalen Ende (10) des frontseitigen Hebels (1) der Tenakulumzange vorzugsweise 20 mm beträgt.

10. Tenakulumzange nach Anspruch 1, wobei der Abstand d₂ des Paars Zinken (16, 17) vom distalen Ende (11) des rückseitigen Hebels (2) der Tenakulumzange in einem Bereich von 10 bis 20 mm enthalten ist.

11. Tenakulumzange nach Anspruch 1, wobei der Abstand d₂ des Paars Zinken (16, 17) vom distalen Ende (11) des rückseitigen Hebels (2) der Tenakulumzange vorzugsweise 15 mm beträgt.

12. Tenakulumzange nach Anspruch 1, wobei der Zinken (14) einen Winkel α von 90 Grad mit dem Zinken (15) bildet, und der Zinken (16) einen Winkel α von 90 Grad mit dem Zinken (17) bildet.

## Revendications

1. Tenaculum pour hystérectomie laparoscopique comprenant un levier antérieur (1) et un levier postérieur (2) montés pivotant dans leur position centrale (3), le levier antérieur (1) et un levier postérieur (2) comportant des extrémités proximales (4, 5), pourvues d'un anneau (6, 7), et des extrémités distales (10, 11), chacune pourvue d'au moins un point (12, 13), les extrémités distales (10, 11) étant incurvées vers l'une et l'autre avec les points (12, 13) se faisant face, **caractérisé en ce que** :
ledit levier antérieur (1) comporte une première paire de dents (14, 15) en forme de V de longueur ℓ dépassant vers l'extérieur symétriquement et orthogonalement par rapport au levier antérieur (1) opposé au levier postérieur (2), et à une distance donnée d₁ de ladite extrémité distale (10) ; et
ledit levier postérieur (2) comporte une seconde paire de dents (16, 17) en forme de V de longueur ℓ dépassant vers l'extérieur symétriquement et selon un angle aigu β par rapport au levier postérieur (2) opposé au levier antérieur (1), et à une distance donnée d₃ de l'extrémité distale (11).

2. Tenaculum selon la revendication 1, dans lequel ledit angle aigu β formé par ladite seconde paire de dents (16, 17) par rapport au levier postérieur (2) est compris entre 45 et 55 degrés.

3. Tenaculum selon la revendication 2, dans lequel ledit angle aigu β formé par ladite seconde paire de dents (16, 17) par rapport au levier postérieur (2) est de préférence de 50 degrés.

4. Tenaculum selon la revendication 1, dans lequel les dents (14, 15 et 16, 17) des première et seconde paires de dents ont une forme cylindrique et une extrémité avec une partie terminale sphérique (18).

5. Tenaculum selon la revendication 4, dans lequel les dents (14, 15 et 16, 17) ont un diamètre D₁ de 2 mm et leurs parties terminales sphériques (18) ont un diamètre D₂ de 2,7 mm.

6. Tenaculum selon la revendication 1, dans lequel la longueur ℓ des dents est comprise entre 7 et 10 mm.

7. Tenaculum selon la revendication 1, dans lequel la longueur ℓ des dents est de préférence de 8 mm.

8. Tenaculum selon la revendication 1, dans lequel la distance d₁ de la paire de dents (14, 15), à partir de l'extrémité distale (10) du levier antérieur (1) du tenaculum, est comprise entre 15 et 25 mm.

9. Tenaculum selon la revendication 8, dans lequel la distance d₁ de la paire de dents (14, 15), à partir de l'extrémité distale (10) du levier antérieur (1) du tenaculum, est de préférence de 20 mm.

10. Tenaculum selon la revendication 1, dans lequel la distance d₂ de la paire de dents (16, 17), à partir de l'extrémité distale (11) du levier postérieur (2) du tenaculum, est comprise entre 10 et 20 mm.

11. Tenaculum selon la revendication 1, dans lequel la distance d₂ de la paire de dents (16, 17), à partir de l'extrémité distale (11) du levier postérieur (2) du tenaculum, est de préférence de 15 mm.

12. Tenaculum selon la revendication 1, dans lequel la dent (14) forme un angle α de 90 degrés avec la dent (15), et la dent (16) forme un angle α de 90 degrés avec la dent (17).
